(19) Europäisches Patentamt
European Patent Office
Office européen des brevets

(11) **EP 4 549 456 A1**

(12) **EUROPEAN PATENT APPLICATION**
published in accordance with Art. 153(4) EPC

(43) Date of publication:
07.05.2025 Bulletin 2025/19

(21) Application number: 23830322.6

(22) Date of filing: 28.06.2023

(51) International Patent Classification (IPC):
*C07K 14/78* (2006.01)    *C12N 15/12* (2006.01)
*C12N 15/81* (2006.01)    *C12N 1/19* (2006.01)
*A61K 38/39* (2006.01)    *A61K 8/65* (2006.01)
*A61P 29/00* (2006.01)

(86) International application number:
PCT/CN2023/103141

(87) International publication number:
WO 2024/002149 (04.01.2024 Gazette 2024/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.06.2022 CN 202210739807

(71) Applicants:
• **Bloomage Biotechnology Corporation Limited**
Jinan, Shandong 250101 (CN)
• **Bloomage Biotech (Tianjin) Co., Ltd.**
Tianjin 300270 (CN)

(72) Inventors:
• **ZHOU, Wei**
Jinan, Shandong 250101 (CN)

• **LU, Yuting**
Jinan, Shandong 250101 (CN)
• **XU, Guoxia**
Jinan, Shandong 250101 (CN)
• **ZHANG, Jing**
Jinan, Shandong 250101 (CN)
• **LIU, Mingming**
Jinan, Shandong 250101 (CN)

(74) Representative: **Abel & Imray LLP**
**Westpoint Building**
**James Street West**
**Bath BA1 2DA (GB)**

Remarks:
The complete document including Reference
Table(s) and the Sequence Listing(s) can be
downloaded from the EPO website

(54) **RECOMBINANT TYPE-III COLLAGEN AND METHOD FOR PREPARING SAME**

(57) Provided are a recombinant type-III collagen and a method for preparing same and use thereof. The amino acid sequences of the recombinant type-III collagen are set forth in SEQ ID NOs: 1, 2, and 3.

EP 4 549 456 A1

**Description**

**TECHNICAL FIELD**

This application belongs to the field of biotechnology. Specifically, this application relates to a recombinant type-III collagen and a method for preparing the same.

**BACKGROUND**

Collagen is a biopolymer that is widely present in animal connective tissues, accounting for approximately 25% to 30% of the total protein in the body. It is the functional protein with the widest distribution and the largest mass fraction in mammals. Because of its good biocompatibility, biodegradability, low immunogenicity and biological activity (for example, it can promote cell proliferation, differentiation, migration for repair and hemostasis), it is widely used in cosmetics, food, medicine, tissue engineering and other fields.

The current main collagen preparation methods include animal-derived extraction methods and genetic engineering methods. The extraction of animal-derived collagen is mainly industrialized through acid and enzymatic methods, with relatively lower costs. However, there are issues such as animal-derived disease infections, the possibility of immune rejection or allergic reactions caused by heterologous collagen, and production capacity limitations. Genetic engineering technology produces recombinant collagen with single components, high safety, and controllable production process. In view of the broad application prospects of collagen, especially the large demand for high-quality collagen in high-end medical materials, health products and other fields, recombinant expression of collagen by genetic engineering technology has become a very significant research direction.

Genetic engineering involves different expression systems such as *Escherichia coli,* yeast, insect cells, mammalian cells, and genetically modified crops. Expression systems such as mammalian cells and insect cells have high costs and long cycles, making it difficult to meet the needs of industrialization. In contrast, microbial fermentation for the production of recombinant collagen has lower costs, shorter cycles, easier cultivation, and is easier to commercialize.

Common drawbacks in bacterial expression systems include: the pyrogen produced makes it difficult to apply the expression product in clinical applications; the target protein is expressed in the form of inclusion bodies, making product purification difficult; the post-translational processing and modification system of the prokaryotic expression system is imperfect, and the biological activity of the expression product is poor. Using the *Pichia pastoris* expression system can avoid these shortcomings, and can perform certain post-translational modifications on the translated proteins (including disulfide bond formation and glycosylation, etc.), which is a strong support realization of protein biological functions. The establishment of an expression system using *Pichia pastoris* has the advantages of large-scale industrial production in microbial expression system, such as high-density fermentation production, low culture costs, and high protein expression levels. Moreover, it can be secreted extracellularly, avoiding impurity proteins caused by bacterial lysis. At the same time, its cell wall components do not contain endotoxins or peptidoglycans.

SUMMARY

In view of the above problems existing in the prior art, this application uses genetic engineering technology to recombinantly express a new type-III collagen, which can avoid viral risks and rejection reactions, and has a definite molecular weight. The production process conditions are mild, which is conducive to maintaining the biological activity of the protein. At the same time, this application provides a method for preparing the recombinant type-III collagen.

Specifically, this application relates to the following aspects:

1. A recombinant type-III collagen, wherein the recombinant type-III collagen is any one of the following A1)-A3):

A1) a protein, consisting of the amino acid sequence shown in SEQ ID NO. 1, SEQ ID NO. 2 or SEQ ID NO. 3;
A2) a protein related to the recombinant type-III collagen obtained by substituting and/or deleting and/or adding one or several amino acid residues to the amino acid sequence shown in A1); and
A3) a protein that has more than 85% identity with A1) or A2) and is related to the recombinant type-III collagen.

2. A nucleic acid molecule, wherein the nucleic acid molecule encodes the recombinant type-III collagen according to item 1.

3. The nucleic acid molecule according to item 2, wherein the nucleic acid molecule is any one of the following C1) or C2):

C1) a DNA molecule with nucleotide sequence shown in SEQ ID NO. 4, SEQ ID NO. 5 or SEQ ID NO. 6;

C2) a DNA molecule that has more than 85% identity with the DNA molecule shown in C1) obtained by modifying and/or substituting and/or deleting and/or adding one or several nucleotides to the nucleotide sequence shown in SEQ ID NO. 4, SEQ ID NO. 5 or SEQ ID NO. 6, and has the same function.

4. A vector, wherein the vector comprises the nucleic acid molecule according to item 2.

5. A host cell, wherein the host cell comprises the collagen according to item 1 or the nucleic acid molecule according to item 2 or the vector according to item 4.

6. The host cell according to item 5, wherein the host cell is a prokaryotic cell or a eukaryotic cell.

7. The host cell according to item 6, wherein the eukaryotic cell is *Pichia pastoris.*

8. A method for preparing the recombinant type-III collagen according to item 1, wherein the method includes the following steps:

using the host cell according to any one of items 5-7 to express the recombinant type-III collagen, which is then separated and purified.

9. Use of the recombinant type-III collagen according to item 1, or the recombinant type-III collagen encoded by the nucleic acid molecule according to item 2 or 3, or the recombinant type-III collagen produced by the host cell according to any one of items 5-7 in the preparation of food, a cosmetic or a medicine and medical equipment product.

10. A composition, wherein the composition comprises the recombinant type-III collagen according to item 1, or the recombinant type-III collagen encoded by the nucleic acid molecule according to item 2 or 3, or the recombinant type-III collagen produced by the host cell according to any one of items 5-7.

11. Use of the composition according to item 10 in the preparation of food, a cosmetic or a medicine and medical equipment product.

12. Use of the recombinant type-III collagen according to item 1 or the composition according to item 10 in promoting cell proliferation, maintaining cell activity, cell differentiation or cell migration.

13. Use of the recombinant type-III collagen according to item 1 or the composition according to item 10 for anti-inflammatory purposes.

14. The use according to item 12 or 13 for non-therapeutic purposes.

15. Use of the recombinant type-III collagen according to item 1 or the composition according to item 10 in the preparation of a product for promoting cell proliferation, maintaining cell activity, cell differentiation or cell migration.

16. Use of the recombinant type-III collagen according to item 1 or the composition according to item 10 in the preparation of an anti-inflammatory product.

17. An anti-inflammatory method, including administering the recombinant type-III collagen according to item 1 or the composition according to item 10 to a subject in need thereof.

18. A method for promoting cell proliferation, maintaining cell activity, cell differentiation or cell migration, including administering the recombinant type-III collagen according to item 1 or the composition according to item 10 to a subject in need thereof.

In order to improve the expression level of *Pichia pastoris* as much as possible, this application adopts the following strategies: 1) optimizing the codons encoding various amino acids of recombinant type-III collagen completely to the preferred codons of *Pichia pastoris* to facilitate the expression of the recombination type-III collagen; and 2) optimizing high-density fermentation conditions and processes to explore its high expression potential.

Beneficial effects

In this application, the active amino acid sequence of the recombinant type-III collagen is spliced, and the corresponding base sequence is codon-optimized according to the codon preference of *Pichia pastoris* for expressing heterologous proteins. After strain screening and parameter optimization, the total protein concentration of the fermentation broth supernatant is increased by 50% compared with that before optimization.

The *Pichia pastoris* expression system used in this application does not contain endotoxin, has low production cost and high protein expression. The amino acid composition of the recombinant type-III collagen produced by it is consistent with the $\alpha$1 chain of natural collagen. It will not cause immune rejection when applied to the human body. It can be widely used in the fields of food, cosmetics, health products, and medicine and medical equipment products, and can be produced on an industrial scale. Compared with commercially available collagen products, its cell proliferation, migration and other effects are better.

The recombinant type-III collagen of the application can also significantly promote cell adhesion. Cell adhesion is a dynamic process and the most basic life activity of cells. Therefore, the recombinant type-III collagen of the application plays a key role in promoting cell proliferation, maintaining cell activity, cell differentiation and cell migration. In addition, the recombinant type-III collagen of the application also has anti-inflammatory effects.

## BRIEF DESCRIPTION OF THE DRAWINGS

Figure 1 is a plasmid profile of the recombinant expression vector pPIC9K-COL in Example 2.

Figure 2 is an SDS-PAGE electrophoresis graph of the fermentation supernatant of the recombinant type-III collagen in Example 5.

Figure 3 is a graph showing the results of collagen promoting cell proliferation in Example 6. Different letters "a", "b", "c", and "d" represent significant differences between data, $p < 0.05$.

Figure 4 is a graph showing the results of collagen cell migration in Example 7.

Figure 5 is a graph showing the cell migration rate of collagen in Example 7. Different letters "a", "b", "c", "d", and "e" represent significant differences between data, $p < 0.05$.

Figure 6 shows the adhesion effect of the recombinant type-III collagen on the HaCaT cell model.

Figure 7 shows the anti-inflammatory effect of the recombinant type-III collagen on the HaCaT cell model.

## DETAILED DESCRIPTION

The present application will be described in detail below with reference to the accompanying drawings and specific embodiments. It should be understood that the following examples are only used to illustrate the present application and are not intended to limit the scope of the present application.

Although specific terms are used herein, they are used in a general and descriptive sense only and not for purposes of limitation. Unless otherwise defined, all technical and scientific terms used herein have the same meaning as commonly understood by one of ordinary skill in the art to which the subject matter disclosed belongs.

The techniques and procedures described or referenced herein are conventional methods that are generally well known and frequently used by those skilled in the art.

In one aspect, the present application provides a recombinant type-III collagen.

In a specific embodiment, the amino acid sequence of the recombinant type-III collagen is shown in SEQ ID NO. 1.

In a specific embodiment, the amino acid sequence of the recombinant type-III collagen is shown in SEQ ID NO. 2.

In a specific embodiment, the amino acid sequence of the recombinant type-III collagen is shown in SEQ ID NO. 3.

In the above specific embodiments, recombinant protein refers to the protein obtained by using recombinant DNA or RNA technology to obtain a recombinant vector connected with a gene fragment that can be translated into the target protein, and then transferring it into a host cell that can express the target protein to express specific recombinant protein molecules. In vitro recombinant protein expression systems mainly include the following: 1. prokaryotic cell expression systems, such as E. *coli;* eukaryotic cell expression systems, such as yeast; mammalian cell expression systems, such as CHO cells, and HEK293; and insect cell expression systems. Amino acid sequence refers to the order in which amino acids are connected to form a peptide chain (or polypeptide). Depending on the structure of the amino acids and the way they are linked together, the amino acid sequence can only be read in one direction and form a peptide in a specific form. All amino acids have a regular structure, comprising one carbon flanked by one amino NH3, and one carboxyl COOH. A peptide bond is formed between the amino group of one amino acid and the carboxyl group of another amino acid to form an amino acid sequence. Recombinant collagen refers to collagen prepared through genetic recombination technology, wherein the DNA or RNA encoding collagen is usually inserted into a suitable expression vector and transformed into host cells to express collagen molecules . The DNA or RNA is inserted into the host chromosome by homologous recombination or other means known in the art, and thus used to transform the host cell to produce the protein. Human collagen is divided into more than 28 types of collagen according to different tissue locations, physiological functions, and molecular structures. The most studied are type-I, type-II, and type-III collagen. Among them, type-I collagen and type-III collagen are the two most abundant collagen components in the skin. Type-I collagen is in the form of bundles, supporting the skin structure and maintaining skin toughness; while type-III collagen is more like a fine network, scattered around type-I, tightly meshing dermal cells and water, determining the elasticity and smoothness of the skin. Type-I and type-III collagen are mainly found in connective tissues such as skin, tendons, ligaments, and blood vessels, forming an extracellular matrix network structure that supports organs and protects the body. It is also related to cell attachment and cell migration. 99% of commercially available collagen is animal-derived collagen extracted from animal tissues such as pig skin, cowhide, donkey skin, fish skin, fish scales, etc. Compared with human collagen, there are fundamental differences in the gene sequence and it is a non-homologous substance. After entering the human body, it will be blocked by the immune layer of the skin and cannot penetrate deep into the muscle layer and dermis layer. The gene sequence of the above recombinant type-III collagen is highly consistent with the gene sequence of human type-III collagen and has good tissue compatibility.

Another aspect of the application provides a nucleic acid molecule.

In a specific embodiment, the nucleic acid molecule encodes recombinant type-III collagen. The sequence of the nucleic acid molecule is shown in SEQ ID NO. 4.

In a specific embodiment, the nucleic acid molecule encodes recombinant type-III collagen. The sequence of the nucleic acid molecule is shown in SEQ ID NO. 5.

In a specific embodiment, the nucleic acid molecule encodes recombinant type-III collagen. The sequence of the nucleic acid molecule is shown in SEQ ID NO. 6.

In the above specific embodiments, nucleic acid molecules are the general terms of deoxyribonucleic acid (DNA) and ribonucleic acid (RNA), which are biological macromolecular compounds polymerized from many nucleotide monomers and are one of the most basic substances of life. Nucleotide sequence refers to the order of bases in DNA or RNA. The nucleic acid molecule comprises cDNA. In some cases, the nucleic acid molecule can be modified for use in the vector of the present application, such as for codon optimization. In some cases, sequences can be designed to contain terminal restriction site sequences for the purpose of cloning into a vector. Nucleic acid molecules can be obtained from a variety of sources, such as by polymerase chain reaction (PCR) amplification of encoding nucleic acids within or isolated from one or more given cells.

Another aspect of the present application provides a vector comprising the above nucleic acid molecule.

In a specific embodiment, a vector refers to a self-replicating DNA molecule that transfers DNA fragments (target genes) to recipient cells in genetic engineering recombinant DNA technology. As the most commonly used and simplest vector in genetic engineering, it must include three parts: genetic marker gene, replication region, and target gene. In addition to the commonly used E. *coli* plasmid vectors, many other artificially constructed plasmid vectors suitable for microorganisms, yeasts, plants, etc. have also been developed. Vectors include, but are not limited to: single-stranded, double-stranded or partially double-stranded nucleic acid molecules; nucleic acid molecules containing one or more free ends or without free ends (e.g., circular); nucleic acid molecules containing DNA, RNA, or both; and other polynucleotide species known in the art. One type of vector is a "plasmid," which refers to a circular double-stranded DNA loop into which additional DNA segments can be inserted, such as by standard molecular cloning techniques. Certain vectors are capable of autonomous replication in the host cells into which they are introduced (e.g., bacterial vectors with bacterial origins of replication and episomal mammalian vectors). Other vectors (e.g., non-episomal mammalian vectors) integrate into the host cell's genome upon introduction into the host cell, thereby replicating with the host genome. In addition, certain vectors can direct the expression of target genes. Such vectors are referred to herein as "expression vectors." The recombinant expression vector may comprise a form suitable for expression of the nucleic acid in a host cell, which means that the recombinant expression vector includes one or more regulatory elements, which may be selected based on the host cells for expression and operably linked to the nucleic acid sequence to be expressed. The vectors are recombinant expression vectors pPIC9K-COL1, pPIC9K-COL2, and pPIC9K-COL3.

Another aspect of the present application provides a host cell comprising the above vector.

In a specific embodiment, a host cell refers to any cell type that is susceptible to transformation, transfection, transduction, etc., with the nucleic acid construct or expression vector comprising the polynucleotide of the present application. "Host cell" encompasses any descendant of a parent cell that is not identical to the parent cell due to mutations caused by the replication process. The host cell can be any cell useful in the production of recombinant humanized collagen of the present application. To produce recombinant collagen, the nucleic acid encoding the recombinant collagen can be isolated and inserted into one or more vectors for further cloning/or expression in host cells. Such nucleic acid can be readily isolated and sequenced using conventional techniques (e.g., by using oligonucleotide probes capable of specifically binding to the gene encoding recombinant collagen). The host cell refers to a cell into which exogenous nucleic acid has been introduced, including the progeny of such cells. Host cells include transformants and transformed cells, including primary transformed cells and progeny derived therefrom, regardless of passage number. The descendant may not be identical in nucleic acid content to the parent cells, but may contain mutations. Methods for introducing vectors into host cells are well known. For example, electroporation is used to introduce vectors into host cells. The methods may also be transfection, microinjection technology, gene gun technology, liposome-mediated method, etc. The host cell is a prokaryotic cell or a eukaryotic cell. The host cell is selected from any one of *Pichia pastoris, Saccharomyces cerevisiae, Escherichia coli,* and *Bacillus subtilis.* Preferably, the prokaryotic cell is *Escherichia coli;* preferably, the eukaryotic cell is *Pichia pastoris.*

Another aspect of the present application provides a method for preparing recombinant type-III collagen, including the following steps: using the above host cells to express the recombinant type-III collagen, which is then separated and purified.

In a specific embodiment, the use of host cells for expression refers to culturing the host cells, and the culture medium and culture conditions are well known to those skilled in the art. This application does not impose any restrictions on the expression method, which can be confirmed as needed. For example, the expression method is induced expression. The separation and purification methods include salting out method, ultrafiltration method, affinity chromatography method, gel filtration chromatography method, chromatography method, acid-base precipitation method and membrane separation method, preferably ultrafiltration method.

On the other hand, the present application provides use of the above recombinant type-III collagen, or the recombinant type-III collagen encoded by the above nucleic acid molecule, or the recombinant type-III collagen produced by the above host cells in the preparation of food, cosmetics, health care products or medicine and medical equipment products.

Another aspect of the application provides a composition. The composition comprises the above recombinant type-III collagen, or the type-III collagen encoded by the above recombinant nucleic acid molecule, or the recombinant collagen produced by the above host cell.

In a specific embodiment, the composition is used as a food, cosmetic, health care products or medicine and medical equipment product.

Another aspect of the present application provides use of the above recombinant type-III collagen or the above composition in promoting cell proliferation, maintaining cell activity, cell differentiation or cell migration.

In a specific embodiment, the amino acid sequence of the recombinant type-III collagen is shown in SEQ ID NO. 1. In a specific embodiment, the amino acid sequence of the recombinant type-III collagen is shown in SEQ ID NO. 2. In a specific embodiment, the amino acid sequence of the recombinant type-III collagen is shown in SEQ ID NO. 3. In a specific embodiment, the cells are HaCaT cells. In a specific embodiment, the composition comprises the above recombinant type-III collagen, or the recombinant type-III collagen encoded by the above nucleic acid molecule, or the recombinant collagen produced by the above host cell. In a specific embodiment, the use is for non-therapeutic purposes.

Another aspect of the present application provides use of the above recombinant type-III collagen or the above composition in anti-inflammation.

In a specific embodiment, the amino acid sequence of the recombinant type-III collagen is shown in SEQ ID NO. 1. In a specific embodiment, the amino acid sequence of the recombinant type-III collagen is shown in SEQ ID NO. 2. In a specific embodiment, the amino acid sequence of the recombinant type-III collagen is shown in SEQ ID NO. 3. In a specific embodiment, the anti-inflammatory is anti-inflammatory to HaCaT cells. In a specific embodiment, the composition comprises the above recombinant type-III collagen, or the recombinant type-III collagen encoded by the above nucleic acid molecule, or the recombinant collagen produced by the above host cell. In a specific embodiment, the use is for non-therapeutic purposes.

Another aspect of the present application provides use of the above recombinant type-III collagen or the above composition in the preparation of medicine for promoting cell proliferation, maintaining cell activity, cell differentiation or cell migration.

In a specific embodiment, the amino acid sequence of the recombinant type-III collagen is shown in SEQ ID NO. 1. In a specific embodiment, the amino acid sequence of the recombinant type-III collagen is shown in SEQ ID NO. 2. In a specific embodiment, the amino acid sequence of the recombinant type-III collagen is shown in SEQ ID NO. 3. In a specific embodiment, the cells are HaCaT cells. In a specific embodiment, the composition comprises the above recombinant type-III collagen, or the recombinant type-III collagen encoded by the above nucleic acid molecule, or the recombinant collagen produced by the above host cell.

Another aspect of the present application provides use of the above recombinant type-III collagen or the above composition in the preparation of medicine for anti-inflammatory.

In a specific embodiment, the amino acid sequence of the recombinant type-III collagen is shown in SEQ ID NO. 1. In a specific embodiment, the amino acid sequence of the recombinant type-III collagen is shown in SEQ ID NO. 2. In a specific embodiment, the amino acid sequence of the recombinant type-III collagen is shown in SEQ ID NO. 3. In a specific embodiment, the anti-inflammatory is anti-inflammatory to HaCaT cells. In a specific embodiment, the composition comprises the above recombinant type-III collagen, or the recombinant type-III collagen encoded by the above nucleic acid molecule, or the recombinant collagen produced by the above host cell.

Another aspect of the present application provides a method for promoting cell proliferation, maintaining cell activity, cell differentiation or cell migration, including administering the above recombinant type-III collagen or the above composition to a subject in need thereof.

In a specific embodiment, the amino acid sequence of the recombinant type-III collagen is shown in SEQ ID NO. 1. In a specific embodiment, the amino acid sequence of the recombinant type-III collagen is shown in SEQ ID NO. 2. In a specific embodiment, the amino acid sequence of the recombinant type-III collagen is shown in SEQ ID NO. 3. In a specific embodiment, the cells are HaCaT cells. In a specific embodiment, the composition comprises the above recombinant type-III collagen, or the recombinant type-III collagen encoded by the above nucleic acid molecule, or the recombinant collagen produced by the above host cell.

Another aspect of the present application provides an anti-inflammatory method, including administering the above recombinant type-III collagen or the above composition to a subject in need thereof.

In a specific embodiment, the amino acid sequence of the recombinant type-III collagen is shown in SEQ ID NO. 1. In a specific embodiment, the amino acid sequence of the recombinant type-III collagen is shown in SEQ ID NO. 2. In a specific embodiment, the amino acid sequence of the recombinant type-III collagen is shown in SEQ ID NO. 3. In a specific embodiment, the cells are HaCaT cells. In a specific embodiment, the composition comprises the above recombinant type-III collagen, or the recombinant type-III collagen encoded by the above nucleic acid molecule, or the recombinant collagen produced by the above host cell.

In the above specific embodiment, anti-inflammation refers to helping the body resist the occurrence and spread of inflammation. At this time, the tissue defends against the stimulation produced by various damage factors. The pathological process that occurs during defense is an inflammatory response, which can be resisted by recombinant collagen or the composition thereof to prevent inflammation caused by these factors. This process is called anti-inflammatory. Cell proliferation is an important life characteristic of organisms, which refers to the proliferation of cells by division. Single-celled organisms produce new individuals by cell division. Multicellular organisms produce new cells through cell division to replenish aging or dead cells in the body. Cell differentiation refers to the process in which cells from the same source gradually produce cell populations with different morphological structures and functional characteristics. The result is that the cells are different in space, and the same cell is different from its previous state in time. The essence of cell differentiation is the selective expression of the genome in time and space, through the turning on or off of different gene expressions, ultimately producing signature proteins. Cell migration refers to cell crawling, cell shift or cell movement, which refers to the shift of cells after receiving migration signals or sensing the gradient of certain substances. Cell migration is an alternating process in space and time of the extension of pseudopods at the cell head, the establishment of new adhesion, and the contraction of the cell body tail. Cell migration is one of the basic functions of normal cells, a physiological process of normal growth and development of the body, and a common form of movement of living cells. Cell migration is involved in processes such as embryonic development, angiogenesis, wound healing, immune response, inflammatory response, atherosclerosis, and cancer metastasis.

This description is considered sufficient to enable those skilled in the art to implement the application. Various modifications of the application in addition to those shown and described herein will become apparent to those skilled in the art from the foregoing description and fall within the scope of the appended claims.

Example 1: sequence design of recombinant type-III collagen

Using the NCBI (https://www.ncbi.nlm.nih.govl) website to obtain the natural human type-III collagen $\alpha 1$ chain protein (reference sequence number NP_000081), optimization of functional fragment combinations and physicochemical properties such as isoelectric point and protein stability were performed based on the effective sequence of the mature peptide of human type III collagen. Three type-III collagen protein sequences, COL1, COL2, and COL3, were designed. For specific sequences, please see SEQ ID NO. 1, SEQ ID NO. 2 and SEQ ID NO. 3 in the sequence listing.

Example 2: gene synthesis of recombinant type-III collagen

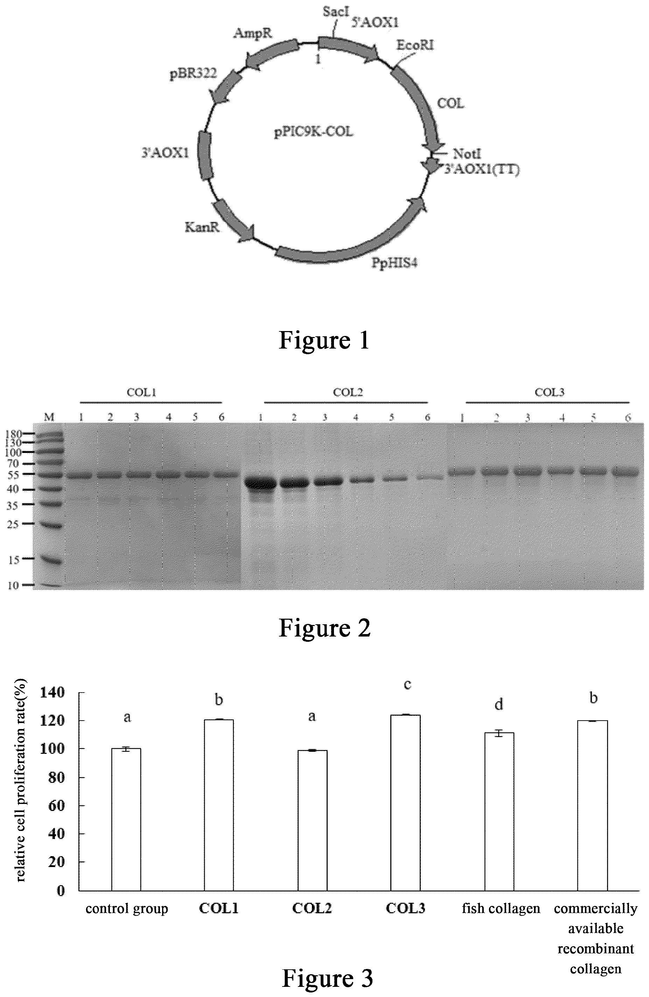

According to the codon preference of *Pichia pastoris* for expressing heterologous proteins, three nucleotide sequences of type-III collagen were designed, artificially synthesized and subcloned into the pPIC9K plasmid. For specific sequences, see SEQ ID NO.4, SEQ ID NO. 5, and SEQ ID NO. 6 in the sequence listing. The whole gene synthesis was completed by Nanjing Genscript Biotech Corporation, and the recombinant plasmid vectors were named pPIC9K-COL1, pPIC9K-COL2, and pPIC9K-COL3 (Figure 1).

Example 3: electrotransformation of *Pichia pastoris*

First, the pPIC9K-COL1, pPIC9K-COL2, and pPIC9K-COL3 recombinant plasmids were linearized with Sac I (purchased from Thermo Fisher Company). The reaction system was as follows:

| component | addition amount ($\mu$L) |
| --- | --- |
| 10×Buffer | 10 |
| recombinant plasmid (200 ng/$\mu$L) | 25 |
| *Sac I* | 5 |
| ddH$_2$O | 70 |
| total system | 100 |

After adding the above reagents, they were mixed well and reacted in a 37°C water bath for 2 hours. Before terminating the reaction, 5 $\mu$L was taken out, and whether the enzyme digestion was completed was detected on agarose gel electrophoresis with a mass concentration of 1.0%. Afterwards, the linearized pPIC9K-COL1, pPIC9K-COL2, and pPIC9K-COL3 plasmids were recovered using a DNA purification kit.

5 μg of pPIC9K-COL1, pPIC9K-COL2, and pPIC9K-COL3 plasmids linearized by Sac I endonuclease and 100 μL of *Pichia pastoris* competent cells were mixed well, transferred to an ice-precooled electrotransformation cup with spacing of 0.2 cm between electrodes, and electroshocked for 4 to 10 milliseconds. 1 mL of ice-precooled 1 M sorbitol solution was added to mix well with the bacteria, and the bacterial was spread on MD (Minimal Dextrase; 1 L MD comprises 13.4 g of YNB, 0.2 g of biotin, 20 g of glucose, and 20 g of agar powder) medium plate, and incubated invertedly at 30°C for 2 to 3 days, until colonies grow on the MD medium plate.

**Example 4: screening of multiple copy insertion recombinants**

The colonies grown on the MD medium plate were inoculated into YPD culture medium plate (yeast extract peptone dextrose; 1L comprises 10g of yeast extract, 20g of tryptone, 20g of glucose, and 20g of agar powder) with G418 concentrations of 0.5g/L, 1g/L, and 2g/L respectively, using sterile toothpicks, and cultured at 30°C. Transformants were obtained through high-concentration antibiotic culture screening. If the *Pichia pastoris* transformant can grow on a plate containing high concentration of G418, it means that the transformant comprises multiple copies of the target gene, that is, multiple recombinant fragments have entered *Pichia pastoris* and been integrated into its chromosomes through homologous recombination. After this screening step, high-copy, high-expression efficiency recombinant *Pichia pastoris* engineered strains can be obtained.

**Example 5: fermentation of recombinant type-III collagen**

The screened transformants were inoculated into 50mL YPD medium to culture with shaking at 30°C, 200rpm, for 24h, and used as primary seeds to be transferred into 280mL YPD medium to culture at 30°C, 200rpm for 24h, and used as secondary seeds to be transferred into a 10L tank containing 3.5L of the main fermentation minimal salt (1L comprises 40g of glycerol, 18.2g of $K_2SO_4$, 26.7mL of $H_3PO_4$, 0.93g of $CaSO_4 \cdot 2H_2O$, 14.9g of $MgSO_4$, and 4.13g of KOH) medium for fermentation. During the fermentation process, the growth temperature was 26°C, the pH value was 5.3, and the dissolved oxygen was controlled at about 20%. When the dissolved oxygen rose sharply, indicating that the glycerol in the minimal salt medium has been exhausted, glycerol with a mass percentage concentration of 50% was added at a flow rate of 15 mL/L/h. The glycerol with a mass percentage concentration of 50% comprised 10 mL/L of the trace element PTM1 (1L comprises 6g of $CuSO_4 \cdot 5H_2O$, 0.08g of NaI, 3g of $MnSO_4 \cdot H_2O$, 0.2g of $Na_2MoO_4 \cdot H_2O$, 0.02g of $H_3BO_3$, 5mL of $H_2SO_4$, 0.5g of $CoCl_2 \cdot 6H_2O$, 20g of $ZnCl_2$, 75g of $FeSO_4 \cdot 7H_2O$, and 0.2g of biotin, and was made by mixing them), and the dissolved oxygen was maintained at 15% to 20%. When the wet bacterial weight of the fermentation broth reached 180-200 mg/mL, the feeding of the fermentation broth was stopped, and starved for 1h. Glycerol was exhausted, and methanol was added to induce fermentation, wherein methanol comprised 10 mL/L PTM1 trace elements. Methanol was added at a rate of 2 mL/L/h for 2 to 3h, and then was added at 10.9mL/L/h for fermentation. By adjusting the rotation speed, tank pressure and ventilation volume to make the dissolved oxygen greater than 15%, fermentation was induced for 96h. The electrophoresis results of the fermentation broth supernatant were shown in Figure 2, wherein Lane M was the protein marker, and the remaining lanes were COL1, COL2 and COL3, respectively.

**Example 6: experiment of recombinant type-III collagen promoting cell proliferation/cytotoxicity**

BALB/C3T3 cells (mouse embryonic fibroblasts) in the logarithmic growth phase (purchased from the Shanghai Cell Bank of the Chinese Academy of Sciences) were inoculated into a 96-well plate at a density of $1 \times 10^5$ cells/mL, with 100 μL per well, and divided into control group and experimental group. They were placed in a carbon dioxide cell culture incubator and routinely cultured for 24h at 37°C, 5% $CO_2$. Solutions of three recombinant type-III collagen sample (COL1, COL2, COL3), fish collagen and commercially available recombinant collagen were prepared using serum-free culture medium (purchased from Jiangsu KeyGEN BioTECH Co., Ltd.). The concentration of each solution was 400 μg/mL, and the solutions were filtered with a 0.22 μm filter to sterilize. After BALB/C3T3 cells were routinely cultured for 24h, the old culture medium was discarded and 100 μL of serum-free culture medium or 100 μL of recombinant type-III collagen sample solution was added, wherein the same amount of serum-free culture medium was added to the control group, and 100 μL of recombinant type-III collagen sample solution was added to the experimental group, 3 parallel samples per group. After continuing to culture for 24h, the culture medium was discarded, and 100 μL of CCK-8 (purchased from Sangon Bioengineering (Shanghai) Co., Ltd.) diluted 10 times in serum-free culture medium was added to each well, and the cells were placed in a cell culture incubator and incubated for another 2h. The relative proliferation rate of cells was detected using the CCK-8 method, and the absorbance was measured with a microplate reader at a wavelength of 450 nm. Relative cell proliferation rate was calculated, wherein relative cell proliferation rate (RGR)% = absorbance value of experimental group/absorbance value of normal control group × 100%.

As shown in Figure 3, in this example, recombinant type-III collagen COL1, COL2, COL3, fish collagen and commercially available recombinant collagen acted on BALB/C3T3 cells for 24h. When the collagen concentration was

400 $\mu$g/mL, the relative cell proliferation rates of COL1, COL3, fish collagen, and commercially available recombinant collagen were significantly higher than those of the control group, increasing by 20.7%, 24.3%, 11.2% and 19.7%, respectively. In addition, the relative cell proliferation rate of COL1 was significantly higher than that of fish collagen, and the relative cell proliferation rate of COL3 was significantly higher than that of fish collagen and commercially available recombinant collagen. The ability of COL2 to promote cell proliferation was not significantly different from that of the control group.

Example 7: detection of cell migration activity of recombinant collagen by scratch method

HacaT cells in the logarithmic growth phase (purchased from the Shanghai Cell Bank of the Chinese Academy of Sciences) were taken as samples, and after routine digestion, they were seeded into the ibidi chamber in a 24-well plate at a density of $3\times10^5$ cells/mL. 70 $\mu$L of cell suspension was added to each of the left and right wells of the chamber, and 400 $\mu$L of serum-free culture medium (purchased from JiangsuKeyGEN BioTECH Biotechnology Co., Ltd.) was supplemented outside the chamber. They were placed in 37°C, 5% $CO_2$ incubator for 24h. The ibidi chamber was taken out, and taken pictures. The culture medium was discarded, and 1 mL of prepared 400 $\mu$g/mL recombinant type-III collagen COL1, COL2, COL3, fish collagen and commercially available recombinant collagen solutions were added, wherein 1 mL of serum-free culture medium was added to the control group. They were observed and taken pictures after continuing to culture for 24h. Image J software (https://imagej.nih.gov/ij) was used to process the pictures of cell migration and analyze the healing rate of the cell scratch area. Data of the initial scratch region area and cell-free blank region area was obtained, and the migration rate was calculated. Cell migration rate (%) = (1-scratch region area/initial scratch region area) $\times$ 100%.

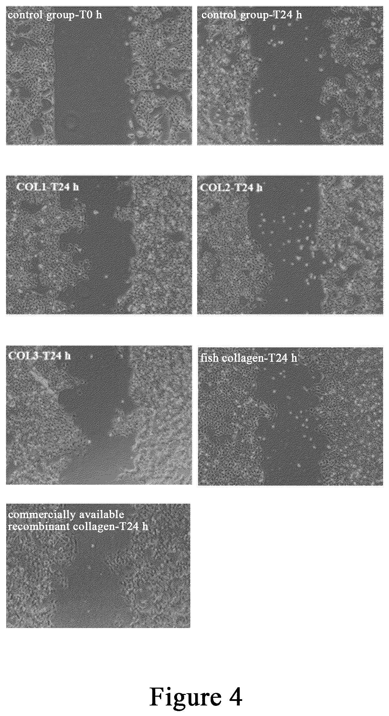

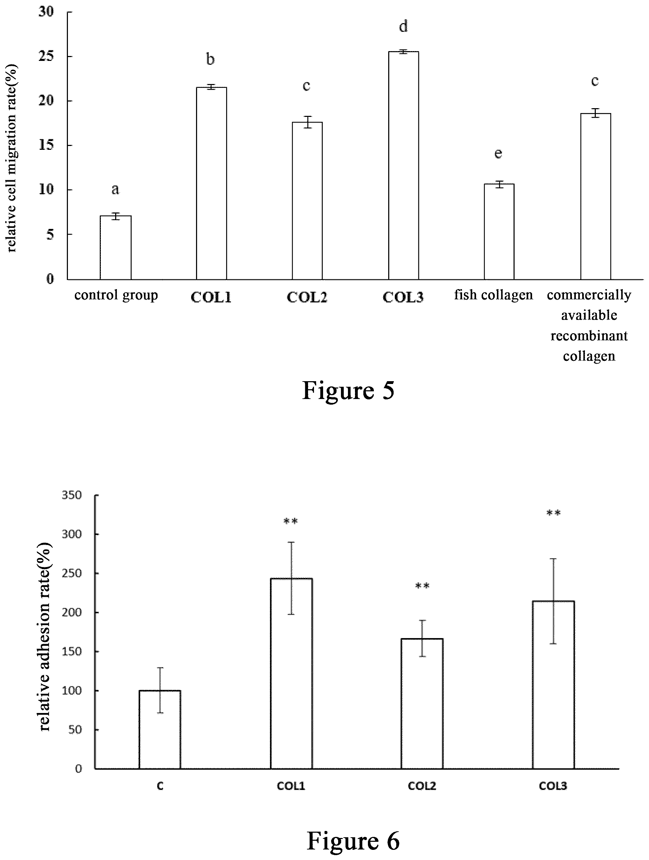

In vitro cell migration experiments simulate the process of cell migration in vivo to a certain extent, directly reflecting the interaction between cells and extracellular matrix and cells under the influence of matrix. Cell migration activity was a more effective indicator of the biological activity of collagen. The higher the migration rate and the faster the speed, the better the biological activity of collagen. The results were shown in Figure 4. Comparing the actual comparison of cell migration taken at 24h (the region inside the two black lines was the initial and post-migration scratch wound regions) and the calculated relative migration rate shown in Figure 5, it can be seen that the migration rates of COL1, COL2 and COL3 were significantly higher than those of the control group, wherein the cell migration rate of COL3 was 3.6 times that of the control group, COL1 was 3.04 times that of the control group, and COL2 was 2.49 times that of the control group. Therefore, COL1, COL2, and COL3 all have strong cell migration activity. Compared with fish collagen and commercially available recombinant collagen, the relative cell migration rates of COL3 and COL1 were significantly higher than fish collagen and commercially available collagen.

**Example 8: evaluation of the adhesion effect of recombinant type-III collagen on HaCaT cell model**

The samples and concentrations to be tested were as follows:

| Group | Final concentration (mg/ml) |
|---|---|
| C | / |
| Recombinant type-III collagen COL1 | 2 |
| Recombinant type-III collagen COL2 | 2 |
| Recombinant type-III collagen COL3 | 2 |

First, the samples were processed. 2 mg/ml recombinant type-III collagen COL1, COL2, and CoL3 were prepared using 1x PBS. The prepared recombinant type-III collagen COL3 was added to a 96-well plate, and 1x PBS was added to the negative control group C. They were placed in a 37°C incubator for 2h, and then the liquid was gently aspirated. They were blocked with 5% BSA in a 37°C incubator for 1h, and washed twice with serum-free medium. Then the cells were inoculated. HaCaT cells were taken and cultured in DMEM medium containing 10% FBS. When the cell density was about 80%, they were digested with 0.05% trypsin, centrifuged at 1000 rpm for 5 minutes, and resuspended in serum-free medium. HaCaT cells resuspended in serum-free medium were added to the wells at a density of $1\times10^5$ cells/well, and incubated at 37°C for 1h. Then, they were washed 4 times with PBS, and MTT was added, and the absorbance was measured 3h later. The absorbance value of MTT was processed and analyzed using the Excel software, and the relative adhesion rate was calculated by the following formula:

$$\text{relative adhesion rate } (\%) = \frac{\text{average of the sample group} - \text{average of the blank group}}{\text{average of the control group} - \text{average of the blank group}} \times 100\%$$

Data was processed was using Graphpad Prism drawing software, and the results were expressed as mean $\pm$ SD. The t test was used for comparison between groups. *P<0.05 means there was a statistical difference, **P<0.01 means there was a significant statistical difference, and ***P<0.001 means there was an extremely significant statistical difference.

The results were shown in Figure 6. Compared with the negative control group C, the relative adhesion rates of 2 mg/ml recombinant type-III collagen COL1, COL2, and CoL3 to HaCaT cells were significantly higher than those of the control group, increasing by 144%, 67%, and 114%, respectively. COL1, COL2, and CoL3 all have significant adhesion-promoting effects.

**Example 9: Evaluation of the anti-inflammatory effect of recombinant type-III collagen on HaCaT cell model**

The samples and concentrations to be tested were as follows:

| Group | Final concentration (mg/ml) |
|---|---|
| C | / |
| Scratch group | / |
| Recombinant type-III collagen COL1 + scratch group | 2 |
| Recombinant type-III collagen COL2 + scratch group | 2 |
| Recombinant type-III collagen COL3 + scratch group | 2 |

First, cells were inoculated. HaCaT cells were cultured in DMEM medium containing 10% FBS. When the cell density was about 80%, they were digested with 0.05% trypsin, centrifuged at 1000 rpm for 5 min, resuspended and counted, inoculated into a 6-well plate, and cultured at 37°C, 5%

$CO_2$. Then the samples were processed, and the medium was aspirated 24h after plating, and samples of corresponding concentrations were added. After the cells grow andfusion, a 200 $\mu$L pipette tip was used to make scratches in the cells, and the cells were washed three times with 1×PBS, and added with serum-free DMEM medium. The supernatant was collected after 6h, and the expression levels of inflammatory factors in each group were detected by ELISA.

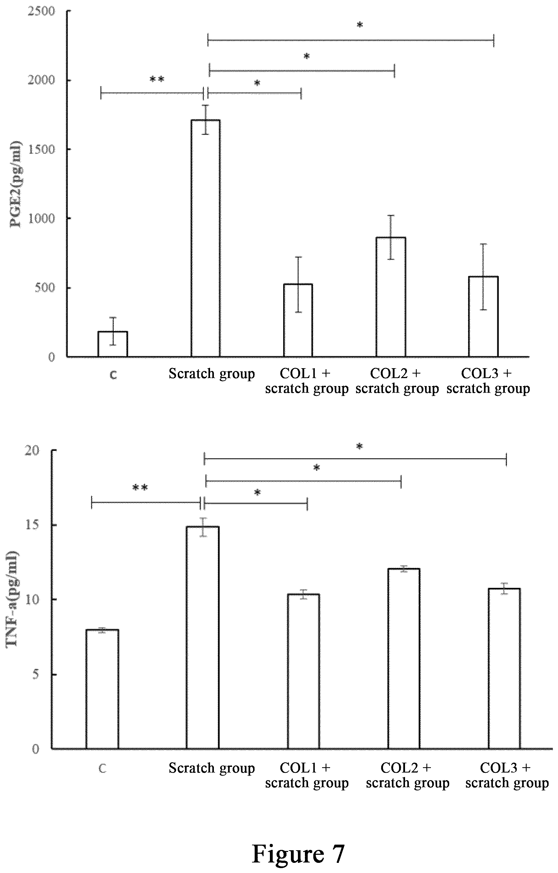

The results were shown in Figure 7. Compared with the negative control group C, the scratch treatment group can cause a significant increase in the inflammatory factor $PGE_2$, increasing by 8.16 times; while the recombinant type-III collagen treatment group COL1 + scratch group, COL2 + scratch group, The COL3 + scratch group can significantly inhibit the scratch-induced increase in $PGE_2$ expression by 69.2%, 49.5% and 66.1%, respectively. Compared with the negative control group C, the scratch treatment group can cause a significant increase in TNF-$\alpha$, increasing by 86.9%; while the recombinant type-III collagen treatment group COL1 + scratch group, COL2 + scratch group, COL3 + scratch group can significantly inhibit the scratch-induced increase in TNF-$\alpha$ expression by 30.3%, 18.9%, and 27.8%, respectively. It shows that recombinant type-III collagen has good anti-inflammatory effects.

Sequence listing:

| Number | Sequence |
|---|---|
| SEQ ID NO.: 1 | EKGETGAPGLKGENGLPGENGAPGAPGERGRPGLPGAAGARGNDGARGSDGQP GPPGPPGTAGFPGSPGAKGEVGPAGSPGSNGAPGQRGEPGPQGHAGAQGPPGPP GINGSPGGKGEMGPAGIPGAPGLMGARGPPGPAGANGAPGLRGGAGEPGKNGA KGEPGPRGERGIPGVPGAKGEDGKDGSPGEPGANGLPGAAGERGAPGFRGPAGP NGIPGEKGPAGERGAPGPAGPRGAAGEPGRDGVPGGPGMRGMPGSPGGPGSDG KPGPPGSQGESGRPGPPGPSGPRGQPGVMGFPGPKGNDGAPGKNGERGGPGGPG PQGPPGKNGETGPQGPPGPTGPGGDKGDTGPPGPQGLQGLPGTGGPPGENGKPG EPGPKGDAGAPGAPGGKGDAGAPGERGPPGLAGAPGLRGGAGPPGPEGGKGAA GPPGPPGAAGTPGLQGMPGER |

(continued)

| Number | Sequence |
|---|---|
| SEQ ID NO.: 2 | GFPEKGETGAPGAPGAAGARGARGQPGPPGPPGTAGFPGSPGAPGQRGEPGPQG HAGEPGAKGEDGKDGSPGEPGANGEKGPAGAPGPAGPRGAAGEPGRDGGPGMR GMPGSPGGPGSDGKPGPPGSQGESGRPGGPGGPGPQGPPGKNGETGPQGPPGPTG PGGDKGDTGPPGPQGENGKPGEPGPKGDAGAPGAPGGKGDAGPPGLAGAAGTP GLQGDKGEGGAPGPRGSPGETGPPGQNGEPGGKGAPGEKGEGGPQGVKGNPGP PGPSGSPGKDGPPGPAGNTGAPGSPGPKGDAGQPGEKGSPGAQGARGPRGSPGP QGESGKPGPPGPQGTAGEPGRDGNPGSDGRDGSPGGKGDRGENGSPGAPGAPGH PGKSGDRGESGPAGPAGAPGPAGSRGAPGPQGPKGETGAAAP |
| SEQ ID NO.: 3 | GAPGLKGENGLPGENGAPGAPGERGAAGARGARGQPGPPGPPGTAGAKGSPGA PGQRGEPGPQGHAGEPGPRGERGAKGEDGKDGSPGEPGANGERGEKGPAGERG APGPAGPRGAAGEPGRDGGPGMRGMPGSPGGPGSDGKPGPPGSQGESGRPGPPG PSGPRGQPGPKGAPGERGGPGGPGPQGPPGKNGETGPQGPPGPTGPGGDKGDTG PPGPQGENGKPGEPGPKGDAGAPGAPGGKGDAGAPGERPPGPEGGKGERGDKG EGGAPGPRGSPGERGETGPPGQNGEPGGKGERGAPGEKGEGGPQGVKGERGAR GNPGPPGPSGSPGKDGPPGPAGNTGAPGSPGPKGDAGQPGEKGSPGAQGARGPR GSPGPQGESGKPGERGPPGPQGTAGEPGRDGNPGSDGRDGSPGGKGDRGENGSP GAPGAPGHPGKSGDRGESGPAGPAGAPGPAGSRGAPGPQGPRGDKGETGERGA AGIKGHRGFPGNPGAP |
| SEQ ID NO.: 4 | GAAAAGGGAGAAACTGGTGCCCCGGGACTTAAGGGAGAGAACGGCCTACCC GGTGAAAATGGAGCCCCGGTGCTCCAGGAGAGCGAGGCCGCCCTGGGTTGC CGGGCGCCGCCGGTGCCCGTGGGAATGATGGCGCCAGAGGTTCGGATGGACA GCCTGGTCCGCCCGGACCGCCTGGCACTGCGGGTTTTCCTGGGTCTCCGGGTG CAAAAGGAGAAGTAGGACCGGCTGGTTCCCTGGTTCCAACGGGGCTCCCGG ACAACGGGGTGAACCTGGACCACAGGGTCACGCGGGCGCCCAGGGGCCTCC AGGACCACCAGGAATCAACGGATCTCCGGGTGGGAAAGGAGAGATGGGCCC CGCAGGTATCCCTGGCGCCCCAGGACTGATGGGCGCAAGGGGGCCGCCGGGG CCCGCAGGTGCGAATGGCGCACCAGGACTCAGAGGTGGGGCAGGAGAGCCA GGTAAGAATGGTGCTAAGGGAGAACCTGGTCCAAGGGGAGAACGAGGGATT CCGGGTGTCCCGGGTGCTAAAGGCGAGGATGGCAAAGATGGGTCCCCGGGA GAGCCCGGGGCAAATGGCCTGCCCGGAGCGGCCGGTGAACGGGGGGCTCCA GGTTTCCGCGGACCTGCGGGTCCCAACGGTATTCCTGGCGAGAAAGGACCCG CTGGGGAACGTGGTGCCCCGGGGCCCGCGGGACCACGGGGCGCCGCAGGGG AACCAGGGAGGGATGGTGTCCCTGGTGGTCCCGGCATGAGGGGAATGCCTGG TTCACCTGGAGGTCCCGGGTCAGATGGCAAACCAGGACCCCCTGGGAGTCAA GGCGAGAGTGGGCGCCCAGGCCCCCCGGCCCATCTGGCCCTCGTGGGCAAC CTGGCGTAATGGGATTTCCCGGTCCAAAGGGAAACGACGGGGCACCGGGAA AAAATGGGGAGCGCGGGGGGGCCAGGGGGGCCCCGGTCCGCAGGGTCCACCGG GGAAGAACGGCGAAACTGGACCCCAGGGTCCCCCTGGACCGACCGGCCCAG GAGGCGATAAAGGTGACACGGGGCCGCCTGGACCGCAAGGGCTTCAAGGAT TACCTGGAACAGGTGGTCCACCAGGGGAAAATGGTAAACCTGGGGAGCCGG GACCGAAAGGAGATGCCGGCGCACCGGGTGCTCCGGGCGGGAAAGGCGACG CGGGCGCTCCAGGTGAACGCGGGCCACCGGGGCTGGCCGGTGCACCCGGGCT AAGAGGCGGGGCGGGCCCCCTGGCCCCGAGGGAGGCAAAGGAGCGGCTGG CCCGCCTGGGCCTCCCGGCGCTGCAGGGACACCCGGCCTCCAGGGTATGCCA GGAGAGCGA |

(continued)

| Number | Sequence |
|---|---|
| SEQ ID NO.: 5 | GGTTTCCCCGAGAAAGGCGAAACCGGAGCACCGGGGGGCCCCGGGAGCTGCG GGCGCTAGGGGCGCTCGTGGCCAACCAGGACCGCCGGGGCCGCCCGGCACA GCTGGATTTCCAGGTTCGCCGGGTGCGCCAGGTCAACGAGGCGAACCCGGCC CCCAGGGGCATGCAGGTGAGCCAGGGGCTAAGGGGGAAGATGGGAAGGACG GGTCACCCGGAGAACCTGGGGCCAACGGGGAAAAGGGACCGGCGGGGGCTC CAGGGCCCGCTGGACCGCGTGGGGCGGCGGGAGAACCCGGTAGGGACGGCG GTCCGGGCATGCGAGGCATGCCCGGTTCTCCGGGTGGGCCTGGCAGTGATGG TAAACCCGGCCCACCAGGTTCTCAAGGAGAGTCCGGCCGGCCTGGCGGGCCA GGGGGACCTGGGCCTCAGGGCCCCCCAGGCAAAAATGGGGAGACAGGCCCC CAAGGCCCACCGGGGCCGACGGGTCCCGGTGGTGATAAAGGAGATACTGGTC CGCCCGGGCCACAGGGTGAGAATGGTAAACCTGGTGAACCAGGCCCAAAGG GAGATGCGGGGGCGCCAGGGGCGCCAGGAGGTAAGGGCGATGCAGGTCCTC CCGGTCTGGCGGGTGCAGCAGGCACACCCGGACTCCAAGGCGACAAAGGAG AGGGGGGGGCACCGGGCCCAAGAGGGTCGCCGGGGGAGACGGGACCTCCGG GTCAGAATGGCGAGCCTGGGGGAAAGGGGGCCCCAGGTGAGAAAGGAGAGG GGGGTCCACAGGGCGTAAAGGGTAACCCCGGCCCTCCGGGGCCTTCTGGCTC TCCGGGAAAAGACGGCCCGCCGGGACCAGCCGGGAATACCGGTGCTCCTGGG AGCCCTGGACCAAAAGGTGACGCTGGCCAACCCGGAGAAAAGGGCAGCCCT GGAGCACAAGGGGCTAGAGGGCCCCGGGGTTCACCGGGACCACAGGGTGAA TCCGGAAAACCCGGACCGCCCGGCCCTCAAGGTACTGCCGGCGAACCTGGGC GCGACGGTAACCCTGGTTCCGATGGCCGGGACGGATCGCCGGGAGGTAAAGG CGATCGCGGAGAAACGGTAGTCCTGGTGCCCCTGGAGCCCCAGGCCACCCC GGGAAGAGTGGAGACCGTGGAGAGAGCGGACCTGCCGGCCCTGCAGGAGCA CCCGGACCTGCCGGTTCAAGGGGTGCCCCTGGCCCACAGGGGCCGAAGGGGG AGACGGGAGCAGCGGCTCCT |
| SEQ ID NO.: 6 | GGGGCACCGGGCCTCAAAGGAGAAAACGGTTTACCTGGAGAAAATGGAGCA CCCGGCGCGCCCGGCGAGCGCGGAGCTGCGGGTGCTCGAGGGGCACGAGGC CAACCCGGACCGCCTGGTCCCCGGGAACGGCTGGCGCGAAGGGATCCCCTG GGGCACCCGGGCAGAGGGGGGAACCAGGCCCGCAGGGGCACGCTGGAGAGC CTGGCCCCAGGGGCGAGCGGGGTGCAAAGGGAGAGGATGGCAAAGATGGCA GTCCCGGGGAGCCAGGGGCTAATGGAGAGAGGAGAGAAGGGCCCAGCCG GTGAAAGAGGGGCCCCGGCCCTGCAGGTCCCCGCGGGGCTGCCGGTGAACC CGGGCGTGATGGTGGTCCCGGCATGAGAGGCATGCCCGGTTCACCGGGTGGT |

(continued)

| Number | Sequence |
|--------|----------|
|  | CCGGGTTCTGACGGAAAACCCGGACCGCCCGGATCTCAGGGAGAGTCAGGTCGTCCTGGTCCGCCAGGGCCCTCGGGACCGCGAGGCCAGCCGGGTCCTAAAGGAGCCCCCGGTGAGCGCGGCGGTCCTGGTGGGCCTGGACCACAAGGTCCCCCAGGTAAGAATGGTGAGACAGGACCCCAGGGCCCGCCTGGCCCCACCGGGCCTGGGGGCGACAAGGGGGATACCGGCCCTCCGGGTCCGCAAGGGGAGAATGGTAAACCAGGGGAGCCAGGCCCAAAAGGCGACGCTGGTGCTCCTGGCGCTCCCGGTGGCAAGGGCGACGCAGGCGCCCCAGGGGAGCGTCCACCAGGACCTGAAGGGGGGAAAGGAGAACGGGGTGATAAGGGGGAAGGCGGCGCGCCAGGTCCTAGGGGTAGCCCGGGAGAAAGAGGCGAAACTGGTCCACCAGGACAAAACGGGGAACCAGGCGGGAAAGGCGAAAGGGGTGCGCCAGGAGAAAAGGGCGAGGGGGGTCCTCAGGGGGTTAAAGGGGAGCGGGGGGCCCGCGGGAACCCCGGACCTCCAGGACCTAGTGGATCGCCCGGGAAGGACGGACCACCTGGGCCTGCCGGGAATACGGGTGCACCTGGATCACCAGGGCCGAAAGGGGATGCCGGCCAACCCGGTGAAAAGGGTTCCCCAGGTGCGCAAGGAGCCCGTGGCCCGCGGGGGTCTCCGGGCCCGCAGGGAGAGTCGGGAAAGCCAGGAGAACGTGGACCGCCTGGGCCACAGGGAACAGCGGGAGAGCCGGGTCGAGACGGAAACCCAGGATCTGATGGGCGAGACGGCAGCCCTGGTGGGAAAGGAGACAGAGGAGAAAATGGCAGTCCTGGAGCCCCGGGCGCTCCCGGCCATCCTGGAAAGAGCGGGGATAGAGGAGAAGCGGGCCTGCGGGTCCGGCAGGCGCTCCGGGTCCCGCCGGTTCCCGCGGGGCACCAGGTCCGCAAGGGCCACGGGGCGACAAGGGAGAGACTGGTGAAAGGGGGGCAGCGGGTATTAAAGGGCATCGAGGCTTCCCCGGCAACCCGGGCGCGCCCC |

## Claims

1. A recombinant type-III collagen, wherein the recombinant type-III collagen is any one of the following A1)-A3):

   A1) a protein, consisting of the amino acid sequence shown in SEQ ID NO. 1, SEQ ID NO. 2 or SEQ ID NO. 3;
   A2) a protein related to the recombinant type-III collagen obtained by substituting and/or deleting and/or adding one or several amino acid residues to the amino acid sequence shown in A1); and
   A3) a protein that has more than 85% identity with A1) or A2) and is related to the recombinant type-III collagen.

2. A nucleic acid molecule, wherein the nucleic acid molecule encodes the recombinant type-III collagen according to claim 1.

3. The nucleic acid molecule according to claim 2, wherein the nucleic acid molecule is any one of the following C1) or C2):

   C1) a DNA molecule with nucleotide sequence shown in SEQ ID NO. 4, SEQ ID NO. 5 or SEQ ID NO. 6;
   C2) a DNA molecule that has more than 85% identity with the DNA molecule shown in C1) obtained by modifying and/or substituting and/or deleting and/or adding one or several nucleotides to the nucleotide sequence shown in SEQ ID NO. 4, SEQ ID NO. 5 or SEQ ID NO. 6, and has the same function.

4. A vector, wherein the vector comprises the nucleic acid molecule according to claim 2.

5. A host cell, wherein the host cell comprises the collagen according to claim 1 or the nucleic acid molecule according to claim 2 or the vector according to claim 4.

6. The host cell according to claim 5, wherein the host cell is a prokaryotic cell or a eukaryotic cell.

7. The host cell according to claim 6, wherein the eukaryotic cell is *Pichia pastoris.*

8. A method for preparing the recombinant type-III collagen according to claim 1, wherein the method includes the following steps:
   using the host cell according to any one of claims 5-7 to express the recombinant type-III collagen, which is then separated and purified.

9. Use of the recombinant type-III collagen according to claim 1, or the recombinant type-III collagen encoded by the nucleic acid molecule according to claim 2 or 3, or the recombinant type-III collagen produced by the host cell according to any one of claims 5-7 in the preparation of food, a cosmetic or a medicine and medical equipment product.

10. A composition, wherein the composition comprises the recombinant type-III collagen according to claim 1, or the recombinant type-III collagen encoded by the nucleic acid molecule according to claim 2 or 3, or the recombinant type-III collagen produced by the host cell according to any one of claims 5-7.

11. Use of the composition according to claim 10 in the preparation of food, a cosmetic or a medicine and medical equipment product.

12. Use of the recombinant type-III collagen according to claim 1 or the composition according to claim 10 in the preparation of a product for promoting cell proliferation, maintaining cell activity, cell differentiation or cell migration.

13. Use of the recombinant type-III collagen according to claim 1 or the composition according to claim 10 in the preparation of anti-inflammatory products.

Figure 1

Figure 2

Figure 3

Figure 4

Figure 5

Figure 6

Figure 7

| INTERNATIONAL SEARCH REPORT | International application No.<br><br>**PCT/CN2023/103141** |
|---|---|

**A.    CLASSIFICATION OF SUBJECT MATTER**

C07K 14/78(2006.01)i；  C12N 15/12(2006.01)i；  C12N 15/81(2006.01)i；  C12N 1/19(2006.01)i；  A61K 38/39(2006.01)i；
A61K 8/65(2006.01)i；  A61P 29/00(2006.01)i

According to International Patent Classification (IPC) or to both national classification and IPC

**B.    FIELDS SEARCHED**

Minimum documentation searched (classification system followed by classification symbols)

IPC: C07K C12N A61K A61P

Documentation searched other than minimum documentation to the extent that such documents are included in the fields searched

Electronic data base consulted during the international search (name of data base and, where practicable, search terms used)

CNABS, WPABS, WPABSC, DWPI, CNTXT, WOTXT, USTXT, EPTXT, EPTXTC, CNKI, PUBMED, ISI web of science, Genebank, EMBL, STN, 万方数据库, WANFANG Database, 百度学术, BAIDU SCHOLAR, 中国专利生物序列检索系统, China Patents Biological Sequence Search System. 胶原蛋白, 功能片段, 连接, 拼接, Type III collagen, Collagen, fragment, Stitching, Connect, Combination, Chimeric, 对SEQ ID NO: 1-6进行序列检索, sequence search for SEQ ID NOs: 1-6.

**C.    DOCUMENTS CONSIDERED TO BE RELEVANT**

| Category* | Citation of document, with indication, where appropriate, of the relevant passages | Relevant to claim No. |
|---|---|---|
| A | CN 110606896 A (JIANGSU YUEZHI BIOLOGICAL MEDICINE CO., LTD.) 24 December 2019 (2019-12-24)<br>see claim 1, and embodiments 1-6, SEQ ID NO: 1 | 1-13 |
| A | CN 114085284 A (XI'AN DENOVO HITH MEDICAL TECHNOLOGY CO., LTD.) 25 February 2022 (2022-02-25)<br>see claims 1-10, and SEQ ID NOs: 1-5 | 1-13 |
| PX | CN 114805551 A (BLOOMAGE BIOTECHNOLOGY CORPORATION LIMITED; BLOOMAGE BIOTECHNOLOGY (TIANJIN) CO., LTD.) 29 July 2022 (2022-07-29)<br>see claims 1-11, and SEQ ID NOs: 1-6 | 1-13 |
| A | US 2006199180 A1 (MACINA ROBERTO A; SALCEDA SUSANA; LIU CHENGHUA; SUN YONGMING; TURNER LEAH R) 07 September 2006 (2006-09-07)<br>see SEQ ID NO: 288 | 1-13 |

☑ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 July 2023** | **30 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/103141**

**C.    DOCUMENTS CONSIDERED TO BE RELEVANT**

| Category* | Citation of document, with indication, where appropriate, of the relevant passages | Relevant to claim No. |
|---|---|---|
| A | EMBL NUMBER. "JB613164"<br>*EMBL*, 11 November 2013 (2013-11-11),<br>    see entire document | 1-13 |

Form PCT/ISA/210 (second sheet) (July 2022)

**INTERNATIONAL SEARCH REPORT**

International application No.

**PCT/CN2023/103141**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

| International application No. |
| --- |
| **PCT/CN2023/103141** |

| Patent document cited in search report | | | Publication date (day/month/year) | Patent family member(s) | | | Publication date (day/month/year) |
| --- | --- | --- | --- | --- | --- | --- | --- |
| CN | 110606896 | A | 24 December 2019 | None | | | |
| CN | 114085284 | A | 25 February 2022 | None | | | |
| CN | 114805551 | A | 29 July 2022 | None | | | |
| US | 2006199180 | A1 | 07 September 2006 | WO | 2004013311 | A2 | 12 February 2004 |
| | | | | WO | 2004013311 | A3 | 04 May 2006 |
| | | | | US | 7678889 | B2 | 16 March 2010 |
| | | | | AU | 2003258127 | A1 | 23 February 2004 |

Form PCT/ISA/210 (patent family annex) (July 2022)